# EUROPÄISCHE PATENTANMELDUNG

(11) **EP 2 578 152 A1**
(43) Veröffentlichungstag der Anmeldung: **10.04.2013**
(21) Anmeldenummer: 12006932.3
(22) Anmeldetag: 05.10.2012
(51) Int. Cl.: A61B 5/11, A61B 5/00, A61B 5/107

(54) **Sprunggelenk-Arthrometer**

(30) Priorität: 07.10.2011 DE 102011115029
(71) Anmelder: ELMAKO GmbH & Co. KG, 76473 Iffezheim (DE)
(72) Erfinder:
(74) Vertreter: Jany und Petersen

(57) **Zusammenfassung**

Die Erfindung betrifft eine Vorrichtung zur Feststellung von Verletzungen von Bändern des menschlichen Sprunggelenks, mit einer Stütze für einen Unterschenkel und mit einer gegenüber dieser Stütze im Wesentlichen in Normalrichtung verschiebbaren Fußstütze. Um hier reproduzierbare Ergebnisse zu erhalten wird vorgeschlagen, die Verschiebbarkeit der Fußstütze über einen motorischen Antrieb zu gewährleisten. Insbesondere wird hierbei eine magnetisch wirkende Kraftbegrenzung zum Einsatz gebracht, die eine unbeabsichtigte Verletzung der Sprunggelenkbänder verhindert.

## Beschreibung

Die Erfindung betrifft eine Vorrichtung zum Feststellen von Verletzungen von Bändern des menschlichen Sprunggelenkes, mit einer Stütze für einen Unterschenkel und mit einer Fußstütze, wobei die Stütze und die Fußstütze im Wesentlichen in Normalrichtung zueinander verschiebbar sind.

Eine Vorrichtung der oben beschriebenen Art wird durch die Firma Blue Bay Medical Inc., Navarre, Florida, USA unter der Bezeichnung "Blue Bay Ankle Arthrometer" angeboten.

Das menschliche Sprunggelenk ist das Verbindungsgelenk zwischen dem Unterschenkel und dem Fuß. Es wird durch eine Reihe von Bändern gehalten, wobei insbesondere die so genannten Außenbänder besonders häufig von Umknickverletzungen betroffen sind, wie Bänderzerrungen, Bänderdehnungen oder Bänderrissen. Es gibt Statistiken, nach denen die Sprunggelenke bei etwa 20 % aller Sportverletzungen betroffen sind.

Um nun festzustellen, ob bei einer derartigen Sportverletzungen eines oder mehrere der Bänder des Sprunggelenks nur gezerrt, gedehnt oder aber gerissen sind, werden üblicherweise Röntgenaufnahmen gemacht. Hierzu wird ein Fuß dann in zwei bzw. vier anatomischen Ebenen geröntgt und später werden noch so genannte gehaltene Aufnahmen angefertigt, bei denen das Gelenk unter einer definierten Krafteinwirkung noch einmal geröntgt wird. Bei unklarem Befund werden häufig auch noch spezielle Schrägaufnahmen angeordnet, bevorzugtermaßen so genanntes Schichtröntgen (CT) oder eine Aufnahme mit der Kernspintomographie (MRT).

All diese Diagnostikverfahren sind aber relativ teuer und aufwändig und bei Röntgenaufnahmen für den Patienten aufgrund der Strahlenbelastung zusätzlich problematisch.

Um gerade derartige Belastungen z. B. durch Röntgenstrahlungen für den Körper auszuschließen, werden stattdessen so genannte Arthrometer vorgeschlagen, mit denen die Reaktion des Sprunggelenks auf Verschiebungen des Fußes gegenüber dem Unterschenkel ermittelt werden, sei es in Richtung nach vorne (d. h. in Richtung des Schienbeins) oder sei es nach hinten (d. h. in Richtung des Wadenbeins).

Bei der oben beschriebenen vorbekannten Vorrichtung wird die Verschiebung des Fußes bewirkt, indem eine Platte, auf der eine Fußstütze den Fuß eines Patienten fixiert, normal zu einer Halterung für den Unterschenkel bewegt wird. Diese Verschiebung erfolgt manuell mittels eines an der Platte befestigten Handgriffs. Dann wird eine sich am Ende der Verschiebung ergebende Kraft gemessen, wobei aus der gemessenen Kraft wiederum Rückschlüsse auf den Zustand des Band-/Halteapparats des Sprunggelenkes möglich sind.

Es hat sich jetzt aber herausgestellt, dass die von den Bändern bzw. dem Halteapparat gelieferte Kraft auch abhängig ist von der Geschwindigkeit, mit der die Platte bzw. die auf dieser befindliche Fußstütze verschoben wird. Es ist dabei anscheinend sowohl relevant, ob die Verschiebung langsam oder schnell erfolgt als auch ob sie gleichmäßig oder ruckelnd aufgebracht wird.

Auch die Größe der Kraft, mit der auf die Platte bei der Verschiebung eingewirkt wird, ist für die Reaktion des Band- und Halteapparates des Sprunggelenkes wesentlich.

Aufgabe der vorliegenden Erfindung ist es daher, eine Vorrichtung wie oben angegeben derart weiterzubilden, dass die angegebenen Einflussgrößen ein geliefertes Ergebnis weniger beeinflussen oder sogar verfälschen können und dass von der Vorrichtung gelieferte Ergebnisse somit reproduzierbar sind.

Diese Aufgabe wird erfindungsgemäß dadurch gelöst, dass die Verschiebung von Fußstütze und Stütze über einen motorisch bewegten Schlitten erfolgt.

Die Erfindung hat den Vorteil, dass die motorische Bewegung des Schlittens mit einer gesteuerten Geschwindigkeit und einer gesteuerte Kraft möglich ist. Damit ist zunächst einmal die Bewegung des Schlittens reproduzierbar und auch die durch die Vorrichtung in Sprunggelenken bewirkten Reaktionen sind demgemäß miteinander zu vergleichen und damit für eine Diagnose geeigneter.

Die Verwendung einer entsprechenden Vorrichtung hat somit den Vorteil, dass die Ergebnisse für einen behandelnden Arzt aussagekräftiger sind, wobei gleichzeitig einem Patienten mehrere Röntgenaufnahmen erspart werden können und somit seine Strahlenbelastung gesenkt werden kann.

Insbesondere können mit einer derartig motorisch bewegten Stütze für den Fuß auch über einen längeren Zeitraum wiederholt miteinander im Hinblick auf die gelieferten Ergebnisse vergleichbare Tests durchgeführt werden, womit ein Heilungsverlauf bei einem verletzten Sprunggelenk kontrollierbar ist.

Bei einer bevorzugten Ausführungsform ist die Fußstütze auf dem Schlitten angebracht und wird mit diesem verfahren. Dies hat praktische Vorteile in der Anwendung. Es ist aber auch möglich, die Stütze am Schlitten zu befestigen und diese zu bewegen.

Bei einer bevorzugten Ausführungsform ist der Schlitten mit einer Kraftmessvorrichtung in Verschieberichtung versehen. Hierdurch ist die vom Schlitten aufgebrachte Kraft und damit auch eine Veränderung dieser Kraft auch während des Verfahrens präzise zu erfassen.

Auch eine Wegmessvorrichtung ist möglich, so dass nicht nur Minimal- oder Maximalwerte sondern ein Kraft/Weg-Verlauf zur Auswertung zur Verfügung steht.

Für eine solche Wegmessvorrichtung ist entweder eine direkte Erfassung der Schlittenposition möglich oder auch eine Inkrementgeber am Antriebsmotor, dessen Impulse erfasst werden und unter Berücksichtigung von Getriebeparametern (z. B. der Steigung einer Antriebsspindel o. ä.) ausgewertet werden.

Damit bei der Benutzung der Vorrichtung die zu untersuchenden Bänder nicht beschädigt werden, ist bezüglich des Antriebs des Schlittens durch den Motor eine Kraftbegrenzung vorgesehen. Diese kann z. B. in der Ankopplung des Schlittens an dem Motor integriert sein oder auch durch eine Drehmomentbegrenzung des Motors realisiert werden.

Es hat sich dabei als besonders effektiv herausgestellt, die Kraftbegrenzung in der Ankopplung als eine magnetische Kupplung auszugestalten. Diese hat den Vorteil bei den unterschiedlichsten Konstellationen sicher auszulösen und auch insbesondere von Reibungseinflüssen relativ unabhängig zu sein.

Weitere Vorteile und Merkmale der Erfindung ergeben sich aus der nachfolgenden Beschreibung eines Ausführungsbeispiels. Dabei zeigt
- Figur 1: Seitenansicht eines Sprunggelenkarthrometers;
- Figur 2: Prinzipskizze eines Sprunggelenkarthrometers in Schnittansicht.

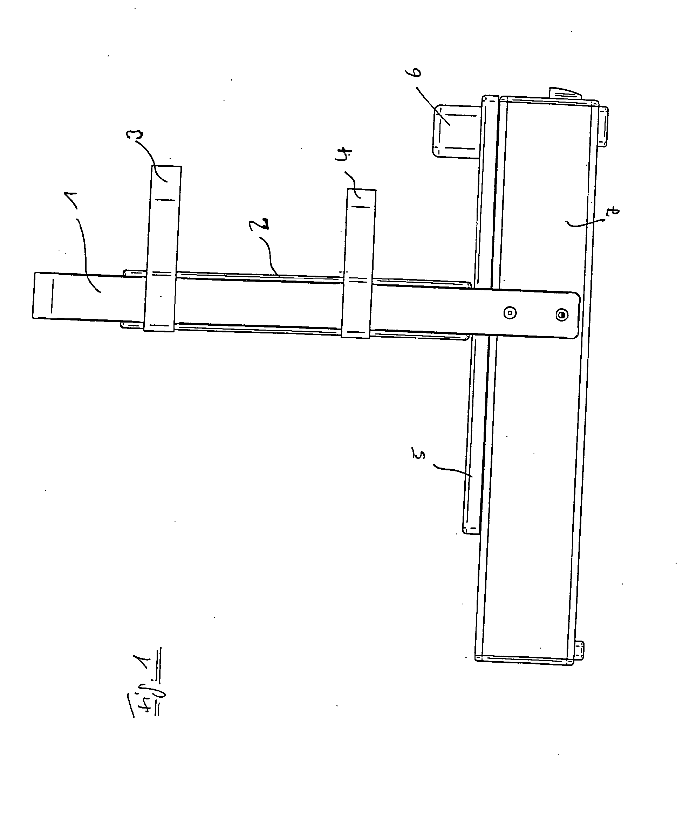

In Figur 1 erkennt man die Seitenansicht eines Sprunggelenkarthrometers.

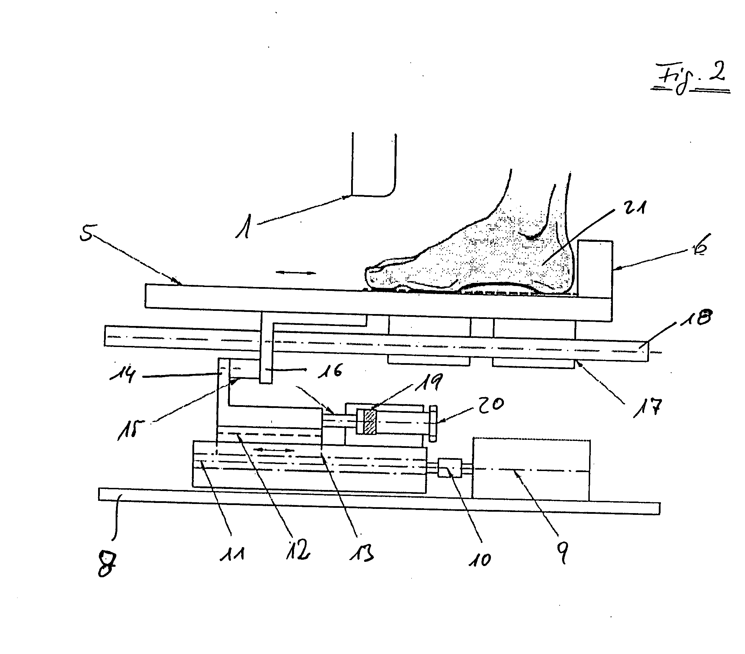

An einer im Wesentlichen vertikal ausgerichteten Stütze 1 wird der Unterschenkel eines Patienten befestigt, wobei dessen Schienbein an ein in die Stütze 1 integriertes Polster 2 anliegt und die Wade des Unterschenkels mit zwei Gurten 3, 4 an der Stütze 1 fixiert wird. Der Fuß des Patienten wird dabei auf eine Platte 5 gesetzt, die an ihrem hinteren in der Figur 1 rechten Ende mit einer Fußstütze 6 versehen ist, über die wie in Figur 2 zu erkennen ist, eine Schubkraft auf den Fuß ausgeübt wird, wenn die Platte 5 in Horizontalrichtung nach vorne (d. h. in der Figur 1 nach links) bewegt wird. Die Bewegung der Platte 5 erfolgt dabei relativ zu dem Gehäuse 7, an dem die Stütze 1 starr befestigt ist. Es wäre erfindungsgemäß auch möglich, die Fußstütze 6 relativ zu der Platte 5 zu bewegen. Allerdings könnten zwischen Fuß und Platte dabei die Ergebnisse verfälschende Reibeffekte auftreten.

In der Figur 2 ist das Innere des Gehäuses 7 zu erkennen.

An dem Gehäuseboden 8 ist ein Elektromotor 9 befestigt, der über eine Kupplung 10 eine Spindel 11 verdreht. Die Kupplung 10 gleicht dabei in bekannter Weise einen axialen Versatz von der Abtriebswelle des Motors 9 und der Spindel 11 aus.

Durch die Verdrehung dieser Spindel 11 wird ein Schieber 12 entlang einer Führung 13 bewegt, in der die Spindel 11 verdrehbar gehalten ist.

Der Schieber 12 trägt einen Kragarm14, an dessen (hier oberen) Ende eine kraftbegrenzende Ankopplung 15 montiert ist, die den Kragarm 14 mit einem Mitnahmearm 16 verbindet. Dieser Mitnahmearm 16 ist fest mit der Platte 5 verbunden. Die Platte 5 kann also über diesen Mitnahmearm 16 verschoben werden, wobei die Platte 5 über Gleitstücke 17 auf einer Führungsstange 18 geführt werden, die im Gehäuse 7 (in hier nicht näher dargestellter Weise) gelagert sind. - Die Kombination aus Schieber 12 und Platte 5 zusammen mit den sie verbindenden Elementen (Kragarm 14, Ankopplung 15 sowie Mitnahmearm 17) bildet somit insgesamt einen motorisch bewegbaren Schlitten.

Die Kraft, die vom Schieber 12 über den Kragarm 14 und den Mitnahmearm 16 auf die Platte 5 ausgeübt wird, kann über einen Drucksensor 19 gemessen werden, wobei über eine Einstellschraube 20 ein gewisser Vordruck auf den Drucksensor 19 ausgeübt werden kann. Dieser Vordruck bewirkt, dass bei Druckaufbau der Drucksensor in einem insbesondere linearen Bereich arbeitet und z. B. Hystereseeffekte ausgeblendet werden können.

Wenn jetzt die Platte 5 nach vorne verfahren wird, so wird der auf dieser Platte stehende Fuß 21 über die Fußstütze 6 nach vorne bewegt, und dabei auch relativ gegenüber dem Schienbein verschoben, das durch die Stütze 1 in seiner Position gehalten wird.

Damit hat man also eine Relativbewegung zwischen dem Gehäuse 7 und dem durch Schieber 12 und Platte 5 gebildeten Schlitten der Vorrichtung.

Damit wird das Sprunggelenk auf Zug nach vorne belastet und die hierbei auftretenden Kräfte werden über den Drucksensor 19 ermittelt und können ausgewertet werden, nämlich dahingehend, ob ein oder mehrere Bänder des Halteapparates des Sprunggelenkes gerissen oder beschädigt sind.

Damit durch die hier beschriebene Vorrichtung die Bänder nicht zusätzlich beschädigt werden, wird über die Ankopplung 15 gewährleistet, dass nur eine Maximalkraft von dem Schieber 12 über seinen Kragarm 14 auf die Platte 5 bzw. die Fußstütze 6 mit dem Mitnahmearm 16 übertragen wird. Der Kragarm 14 schleppt über die Ankopplung 15 den Mitnahmearm 16 nach, wobei die Ankopplung 15 bei Überschreiten einer voreingestellten Kraft auslöst und der Schieber 12 weiterbewegt wird, ohne dass sich die Platte 5 mitbewegt. Damit wird in dem Bewegungsantrieb des Schlittens zwischen Schieber 12 und Platte 5 eine Kraftbegrenzung realisiert.

Besonders geeignet für die Ankopplung 15 ist eine magnetische Halterung. Eine solche kann reibungs- und verschleißunabhängig ausgebildet werden, so dass die damit ausgestattete Vorrichtung auch über längere Zeit eine gleichbleibend hohe Betriebssicherheit gewährleistet.

Alternativ oder auch zusätzlich kann die Kraft des Motors 9 begrenzt werden, beispielsweise über eine Strombegrenzung für diesen.

## Patentansprüche

1. Vorrichtung zum Feststellen von Verletzungen von Bändern des menschlichen Sprunggelenkes, mit einer Stütze (1) für einen Unterschenkel und mit einer Fußstütze (6), wobei die Stütze (1) und die Fußstütze (6) im Wesentlichen in Normalrichtung zueinander verschiebbar sind,
**dadurch gekennzeichnet,**
**dass** die Verschiebung über einen motorischen bewegten Schlitten (5, 12) erfolgt.

2. Vorrichtung gemäß Anspruch 1,
**dadurch gekennzeichnet,**
**dass** die Fußstütze (6) auf dem Schlitten (5, 12) befestigt ist.

3. Vorrichtung gemäß Anspruch 1,
**dadurch gekennzeichnet,**
**dass** der Schlitten (5, 12) mit einer im Wesentlichen in Verschieberichtung wirkenden Kraftmessvorrichtung (19) versehen ist.

4. Vorrichtung gemäß Anspruch 1,
**dadurch gekennzeichnet,**
**dass** der Schlitten (5, 12) mit einer Wegmessvorrichtung für die Verschiebung versehen ist.

5. Vorrichtung gemäß Anspruch 1,
**dadurch gekennzeichnet,**
**dass** in dem Bewegungsantrieb des Schlittens (5, 12) eine Kraftbegrenzung (15) ist.

6. Vorrichtung gemäß Anspruch 5,
**dadurch gekennzeichnet,**
**dass** die Kraftbegrenzung (15) eine magnetische Kupplung ist.
